Europäisches Patentamt

**European Patent Office**

**Office européen des brevets**

(11) Publication number: **0 091 405**
**A2**

(12) **EUROPEAN PATENT APPLICATION**

(21) Application number: **83830057.2**

(22) Date of filing: **15.03.83**

(51) Int. Cl.³: **A 61 F 7/12**
**A 61 F 5/00, A 61 M 31/00**

(30) Priority: **01.04.82 IT 2054982**

(43) Date of publication of application:
**12.10.83 Bulletin 83/41**

(84) Designated Contracting States:
**AT BE CH DE FR GB LI LU NL SE**

(71) Applicant: **Cavina, Enrico**
**Via Mazzini**
**I-56010 Pontasserchio (Pisa)(IT)**

(72) Inventor: **Cavina, Enrico**
**Via Mazzini**
**I-56010 Pontasserchio (Pisa)(IT)**

(74) Representative: **Vannini, Torquato et al,**
**JACOBACCI-CASETTA & PERANI S.p.A. 7 Via Visconti di**
**Modrone**
**I-20122 Milan(IT)**

(54) **Medical appliance for a symptomatic local cryopharmacotherapy of benign ano-rectal ailments, such as haemorrhoids.**

(57) A medical appliance (1) is constituted by an elongate hollow body (2) shaped as a suppository, in which is formed a sealed reservoir (11) filled with coolant. A duct (12) extends longitudinally through the hollow body (2) and, being sealed from the reservoir (11), communicates at one end (4) with the exterior of the body (2) for connection to a source of, for example, a local drug, and at the other end with an irrigation chamber (8) formed in the rounded end (3) of the body (2). The appliance (1) is particularly useful for the cryo-pharmacotherapy of haemorrhoids.

FIG. 1

EP 0 091 405 A2

Medical appliance for a symptomatic local cryo-
pharmacotherapy of benign ano-rectal ailments,
such as haemorrhoids

This invention relates to a medical appliance for a local therapy of benign ano-rectal ailments, such as the home-treatment of haemorrhoids.

With reference to haemorrhoid pathology, it is known that the therapy most widely used in the home is based on the local action of drugs which are readily available on the market. It is equally well known that the benefits achieved are often questionable and frequently non-existent, above all because of the manner in which the drugs are applied. Indeed, disadvantages are recognised which cannot be ignored, such as, for example, a mainly or solely external application, or a solely internal application (suppositories which slide directly into the ampulla) up into the anal passage itself. In addition to this, the temporary nature of the drug-tissue contact is strongly dependent on the mucous secretions and the contaminating passage of the contents of the colon.

For some time, a sedative therapy of haemorrhoids has been developed and become widespread, based on the application of moderate cold (hypothermia of about 10°C) which in a relatively short time of the order of 1 to 2 minutes achieves a noticeable, temporary lowering of the sub-mucous tissue temperature. In this manner, one or more of the following effects can be achieved: antiinflammatory, decongestant, analgesic, anti-sphincteralgesic, anti-edematous, and haemostatic.

This sedative cryotherapy is conducted with a probe of suitable non-toxic plastics material which forms a sealed reservoir filled with cold-retaining gel. Generally, such a probe is cooled beforehand in a refrigerator and is used 2 or 3 times a day.

It is recognised that sedative cryotherapy is useful to the point that it can be used as an alternative to local medication, but the cost of the probe and the obvious need for its replacement after short periods of use (or non-use) is such that the majority of patients still make use of local drug applications.

With specific, but non-exclusive, reference to haemorrhoid pathology, the main object of this invention is to provide, for use in the home, an effective local therapy using hypothermia and drugs simultaneously or, more precisely, an effective cryo-pharmacotherapy.

This and other objects which will become clearer from the following description are achieved by a medical appliance comprising an elongate hollow body shaped substantially as a suppository, which has a rounded end, a sealed reservoir formed in the body and filled with a coolant, and at least one duct extending longitudinally within the body and communicating with the exterior of the body at the opposite end thereof..

In a preferred embodiment of the invention, an irrigation chamber is formed in the hollow body at its rounded end, the chamber being sealed from the coolant reservoir and communicating with the exterior of the body through a plurality of holes formed in the rounded end and with said at least one duct.

Preferably, the duct communicates with the exterior of the elongate hollow body through a plurality of radial passages distributed along the length of the duct.

To advantage, the duct and the hollow body share a common longitudinal wall portion in which the plurality of radial passages is formed.

Further characteristics and advantages of the invention will become clearer from the following description of one embodiment of a medical appliance according to the invention, made with reference to the appended drawings, by way of non-limiting example, in which:

Figure 1 is a partially-sectioned perspective view of a medical appliance according to the invention;

Figure 2 is a longitudinal section of the medical appliance, and

Figure 3 is a cross-section of the medical appliance of Figure 1 on an enlarged scale.

With reference to the drawings, a medical appliance according to the invention, generally indicated 1, has structural characteristics,described below, which make it particularly, but not exclusively, suitable for domestic cryo-pharmacotherapy of haemorrhoids.

The medical appliance includes an elongate hollow body 2 shaped substantially in the form of a conical cylindrical suppository and made from a suitable non-toxic plastics material. The hollow body 2 has one rounded end portion 3, while the other end portion 4 is enlarged and is constituted by a frusto-conical part 5 and a terminal cylindrical part 6. A circular base wall 7 is sealed in the body 2 close to its rounded end 3 by a conventional method, preferably hermetic sealing by ultrasonics, and defines an irrigation chamber 8 with this end. This chamber 8 communicates with the exterior of the body 2 through a plurality of holes 9 formed in the rounded end 3 of the body.

A second circular base wall 10 is hermetically sealed about its periphery in the body 2 in such a position as to constitute a partition between the frusto-conical part 5 and the cylindrical part 6 of the end portion 4 of the body. The cavity in the body 2 defined between the base walls 7, 10 constitutes a reservoir 11 which is sealed hermetically (by means of the ultrasonic method) after being filled with a non-toxic coolant, particularly a liquid gel of the type already widely used commercially, which is free from any toxicity and which retains and redistributes the cold.

By way of example, this liquid may have the following composition:

| | |
|---|---|
| water | 94.2% |
| citric acid | 2.6% |

- 4 -

| sodium chloride | 1.3% |
| β-cellulose (mw 2000-3000) | 1.9% |

the percentages being percentages by weight.

The medical appliance of this invention further includes a duct 12 which extends longitudinally within the body 2 and is structurally independent of the reservoir 11. This duct 12 communicates at one end with the irrigation chamber 8 through a hole formed in the circular base wall 7, to which the end of the duct 12 is sealed. At the other end, the duct 12 extends externally of the circular base wall 10 by a portion 13 which constitutes a connector for connection to any source (phial) of local drugs in the form of liquids, ointments or the like.

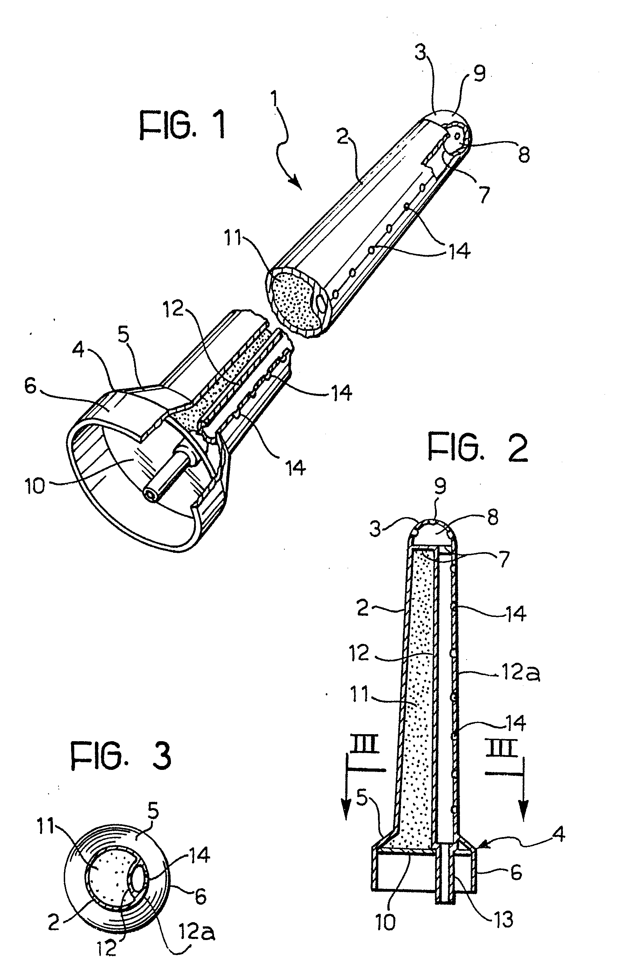

In accordance with a preferred but not exclusive embodiment, the duct 12 and the hollow body 2 share a common wall portion 12a which extends for almost the entire length of the duct (Figures 2 and 3). The duct 12 communicates with the exterior of the body 2 along this portion 12a through a plurality of radial passages 14 arranged in a line longitudinally of the duct.

The medical appliance described above is used in the following manner.

The appliance 1 is normally cooled to between -7 and 25°C in, for example, the freezer of an ordinary refrigerator, for a period of three to four hours. Before use, the body 2 of the medical appliance must be suitably lubricated, for example, oiled with 5% xylocaine gel (which is cooled but not solid), and can then be easily self-administered by the patient for, say, five minutes two or three times a day. During this administration, the local drug may be supplied through the plurality of holes 9, 14 by, for example, a syringe, drop-by-drop melting, or even by gradual and intermittent squeezing when it is constituted by a sufficiently fluid, though cooled, ointment.

The advantages achieved are immediately evident and may be summarised as follows:

- facility of use at home, even by untrained persons or patients;

- possibility of achieving simultaneous cryotherapy and therapy with local drugs which are passed along the whole of the anal passage beyond that at the ampulla;

- possibility of advantageous use for evacuative mini-enemas, account being taken of the evacuative effect produced in the already-dilated anal passage by the medical appliance and the "cold-sedation", and

- ease of manufacture at acceptable costs.

Claims:

1.  A medical appliance (1) for a symptomatic local cryo-pharmacotherapy of benign ano-rectal ailments, such as the home cryo-pharmacotherapy of haemorrhoids, characterised in that it comprises an elongate hollow body (2) shaped substantially as a suppository, which has a rounded end (3), a hermetically-sealed reservoir (11) formed in the body (2) and filled with a coolant, and at least one duct (12) extending longitudinally within the body (2) and communicating with the exterior of the body at the opposite ends thereof.

2.  A medical appliance according to Claim 1, characterised in that it includes an irrigation chamber (8) formed in the hollow body (2) at its rounded end (3), the chamber (8) being sealed from the coolant reservoir (11) and communicating with the exterior of the body (2) through a plurality of holes (9) formed in the rounded end (3) and with said at least one duct (12).

3.  A medical appliance according to Claims 1 and 2, characterised in that the duct (12) communicates with the exterior of the elongate hollow body (2) through a plurality of radial passages (14) distributed along the length of the duct (12).

4.  A medical appliance according to Claim 3, characterised in that the at least one duct (12) and the hollow body (2) share a common portion (12a) of longitudinal wall in which said plurality of radial passages (14) is formed.

5.  A medical appliance according to Claim 4, characterised in that the at least one duct (12) extends externally of the hollow body (2) by a duct portion (13) which constitutes a connector for connection of the duct (12) to a source of a chosen local drug.

FIG. 1

FIG. 2

FIG. 3